(19) Europäisches Patentamt
European Patent Office
Office européen des brevets

(11) Publication number : **0 455 424 A2**

# (12) EUROPEAN PATENT APPLICATION

(21) Application number : 91303758.6

(22) Date of filing : 25.04.91

(51) Int. Cl.⁵ : **C12N 15/85, C12N 15/67, C12N 15/58**

The applicant has filed a statement in accordance with Rule 28 (4) EPC (issue of a sample only to an expert). Accession number(s) of the deposit(s): ATCC CRL-10405 + CRL-10404.

(30) Priority : 02.05.90 US 517983

(43) Date of publication of application :
06.11.91 Bulletin 91/45

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Jenh, Chung-Her**
**7 Dogwood Drive**
**Edison, NJ 08820 (US)**
Inventor : **Law, Ming-Fan**
**12344 Picrus Street**
**San Diego, California 92129 (US)**
Inventor : **Silberklang, Melvin**
**21 Hillside Avenue**
**Englewood, NJ 07631 (US)**
Inventor : **Mark, III, George E.**
**4 Richmond Street**
**Princeton Junction, NJ 07550 (US)**

(74) Representative : **Barrett-Major, Julie Diane et al**
**Merck & Co., Inc. European Patent Department Terlings Park Eastwick Road Harlow Essex CM20 2QR (GB)**

(54) **Mammalian inducible promoter cascade system.**

(57)   The present invention relates to the development of a novel mammalian inducible and cascading gene expression system. Specifically, this invention relates to the development of stable host cell lines, harboring and coexpressing the retroviral transactivator protein (TAT) under the regulation of lacI/lacO, in an inducible expression system in which any cloned gene under transcriptional control of the HIV LTR promoter, can be expressed at a high level in an inducible manner.

EP 0 455 424 A2

## BACKGROUND OF THE INVENTION

One of the major mechanisms for the control of gene expression in eukaryotic cells, analogous to prokaryotic cells, involves the binding of a regulatory protein to a specific DNA sequence that it recognizes. The binding of the regulatory protein to its specific DNA sequence can have a positive or negative effect (activation or repression, respectively) on the transcription of genes.

One of the most thoroughly studied and best understood examples of a protein-DNA interaction that regulates gene transcription is from the lac operon of the bacterium Escherichia coli. Repression of gene expression by the binding of the lac repressor protein to the lac operator DNA sequence blocks transcription from an upstream promoter.

Derepression occurs by the action of inducers, such as isopropyl b-D-thiogalactoside (IPTG), which modify the interaction of the lac repressor protein with the lac operator DNA and allow mRNA transcription to proceed from the promoter.

The lac repressor protein functions as a homotetramer with a subunit molecular weight of 38 kilo Daltons (kD), and has been recombinantly expressed in mammalian cells. It has been shown that lac repressor protein, expressed in mammalian cells, can interact with the lac operator DNA and repress cloned gene expression containing the lac operator in the same cells. Derepression of cloned gene expression was induced by the addition of IPTG to the external medium. This demonstrated that the lac repressor/operator system could function in mammalian cells to control cloned gene expression from an upstream promoter.

Retroviruses, including human immunodeficiency virus (HIV), direct transcription of their viral genes from segments of viral genome known as long terminal repeats (LTR). Transcription from the LTR is activated by the binding of a retroviral gene product, the trans-activator protein (TAT).

TAT is a protein of about l0.5 kD, which is a potent trans-activator of all genes linked to the LTR. TAT, therefore, exerts a strong positive feedback on HIV gene expression. The activation of the LTR promoter has been exploited, to some degree, for the purpose of expressing cloned genes in mammalian cells in culture.

The advent of recombinant DNA technology has made it possible to produce foreign proteins in mammalian cells through the introduction of foreign DNA encoding such proteins. Mammalian expression systems were developed in which the foreign protein was constitutively expressed from an active promoter. This results in the continual expression of the foreign gene. Frequently, the abundant presence of the foreign protein is toxic to the host cell and as a result of constitutive expression, the host cell population may become moribund and perish. Furthermore, the abundant presence of the toxic protein exerts selective pressures on the host cells which can result in the emergence of a cell population containing mutated versions of the foreign DNA, which express grossly modified protein or which have deleted the foreign gene. As a result, commercially useful levels of constitutive expression may never be maintained in the recombinant cell population.

Efforts to combat this shortcoming have resulted in the development of inducible mammalian expression systems which control the expression of the foreign protein. Inducible expression can be achieved by using promoters which are controlled by the presence or absence of a specific regulator. For example, the lac repressor protein blocks expression of the lac genes when it binds to the lac operator DNA. Expression is induced when IPTG is added to the cell culture medium. A foreign gene which is controlled by the lac repressor/operator system would be regulated in the same way as the lac genes. Specifically, in the presence of the lac repressor, the toxic foreign gene product would not be expressed unless IPTG was added to the cells. This system permits the cells to grow to a high density without the toxic effects of the foreign protein. High level expression then can be achieved by adding the inducer, IPTG.

Another means of controlling foreign gene expression involves the use of a promoter which becomes more active in the presence of a specific activator protein. A foreign gene under the control of such a promoter would be expressed at high levels only following the induction of synthesis of the activator. An example of such a promoter/activator system is HIVLTR/TAT. Transcription from the HIV LTR is increased by over l00-fold following the interaction of TAT with the LTR. By controlling the expression of TAT, transcription from the LTR promoter in turn is controlled. Therefore, a foreign gene under the transcriptional control of the HIVLTR will be expressed at high levels only if TAT is present.

## OBJECTS OF THE INVENTION

It is an object of the present invention to provide a mammalian inducible promoter cascade system for high-level expression of cloned genes in an inducible manner. Another object is to provide expression vectors containing multiple lac operators for the controlled expression of the transactivator protein (TAT) of HIV. A further object is to provide stable mammalian cell lines harboring and coexpressing bacterial lac repressor and HIV TAT proteins, in which cell lines the expression of HIV TAT is tightly repressed in the absence of inducers.

Another object is to provide expression vectors containing a TAT-dependent promoter for the inducible expression of inserted DNA sequences in mammalian cells. These and other objects of the present invention will be apparent from the following description.

## SUMMARY OF THE INVENTION

Plasmid pSVlacOSO3TAT-tkHyg was constructed and cointroduced with plasmid pCMVlacI into cultured mammalian cells. Clonal lines were selected in which TAT expression was repressed by the constitutively synthesized lac repressor and induced by IPTG. Recombinant genes for expression were molecularly cloned into plasmids containing the HIV LTR promoter CD23, and were stably introduced into cells harboring both pCMVlacI and pSVlacOSO3TAT-tkHyg. When induced by IPTG, TAT protein activates the CD23 promoter which results in high level expression of the recombinant gene.

## BRIEF DESCRIPTION OF THE DRAWINGS

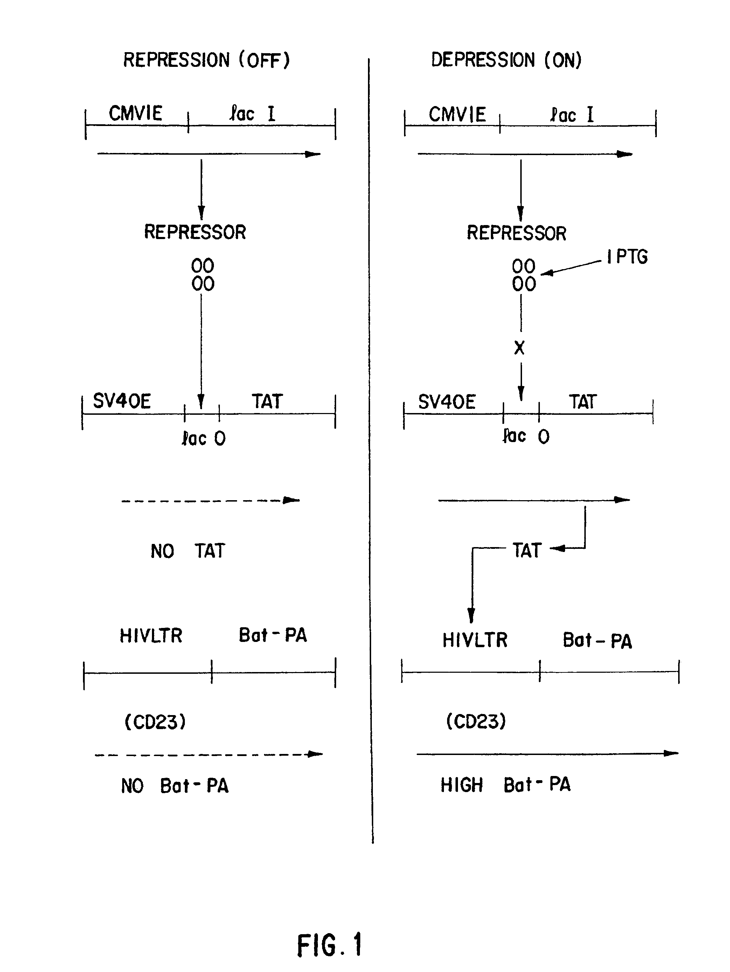

Figure 1 is a diagram of the inducible promoter cascade system. The left side shows the system in the repressed, or off, state. The right side shows the system in the derepressed, or on, state.

Figure 2 is a schematic diagram illustrating the recombinant cloning strategy used to construct pSVlacOSO3TAT.

Figure 3 is a diagram of pSVlacOSO3TAT-tkHyg.

Figure 4 is a diagram of pCD23CAT.

Figure 5 is a diagram of pCD23LacZ.

Figure 6 is a diagram of pCD23Bat-PA.

Figure 7 is a bar graph of a transient CAT expression assay on CIOTAT cells induced with various concentrations of IPTG, showing the percent CAT conversion (bold numbers above bars) and the respective fold increase in CAT expression (numbers in parentheses) over uninduced CIOTAT cells.

Figure 8 is a bar graph of a transient beta-gal expression assay on CIOTAT cells induced with various concentrations of IPTG, showing beta-gal activity (numbers above bars) and the respective fold increase in beta-gal expression (numbers in parentheses) over uninduced CIOTAT cells.

Figure 9 is a series of bar graphs of Bat-PA activity, measured at 2, 3, 4, and 6 days following IPTG induction at various concentrations, on CIOTAT-Bat-PA cells.

## DEFINITION OF TERMS

| Term | Definition |
|---|---|
| Promoter - | A DNA nucleotide sequence specific for the binding of RNA polymerase, which signals the beginning of RNA synthesis. |
| Lac Operator - | A DNA nucleotide sequence specific for the binding of lac repressor which, when bound with lac repressor, blocks RNA synthesis from an adjacent promoter. |
| Lac Repressor - | A protein which binds to the lac operator DNA sequence which, when bound to the lac operator, blocks RNA synthesis from an adjacent promoter. |
| Transcriptionally controlled - | The synthesis of RNA from a sequence of DNA in which the RNA synthesis is signaled by a specific promoter. |
| Transcriptionally linked - | The proximate location of DNA sequence under transcriptional control of a specific promoter. |
| Inducer - | A substance that is capable of activating the RNA synthesis of a structural gene from its transcriptionally linked promoter. |
| Inducible Promoter Cascade - | Promoters arranged in a succession of stages such that the addition of an inducer activates a promoter, the product of which activates another promoter or promoters. |

## DETAILED DESCRIPTION OF THE INVENTION

The present invention is directed to a mammalian inducible promoter cascade system for gene expression. More specifically, it is directed to the use in animal cells of the bacterial lac repressor protein to repress the expression of the TAT gene, via the lac operator DNA sequence. When the inducer IPTG is present, lac repressor protein no longer prevents TAT gene expression, and TAT protein is synthesized. TAT protein, in turn, activates the HIV LTR promoter which causes the expression of any linked gene. This cascade of promoter

3

activation results in the high-level expression of any gene which is transcriptionally controlled by the HIVLTR. In addition, the present invention is directed to the expression, in animal cells, of genes encoding proteins which are toxic to the host cell.

The host cell line utilized in the present invention is the African green monkey kidney-derived CV-IP cell line. CV-IP cells are a fibroblast-like cell line with growth properties and characteristics which are amenable to use in recombinant gene expression systems. It is understood, and is readily apparent to those skilled in the art that a wide variety of commonly used mammalian cell lines are suitable for use in the present invention. Suitable cell lines derived from mammalian species include, but are not limited to, cell lines of human, bovine, porcine, monkey, and rodent origin. Furthermore, suitable cells lines of non-mammalian species include, but are not limited to, cell lines of avian and other higher non-mammalian animal species.

CV-IP cells are grown and maintained in a standard cell culture medium which is commercially available. A suitable, commercially available cell culture medium is Dulbecco's modified Eagle's minimal essential medium (DME) supplemented with heat inactivated newborn calf serum, at a concentration of 10%. The DME and newborn calf serum are available from GIBCO, Gaithersburg, MD. CV-IP cells are typically maintained according to tissue culture techniques which are standard in the art. A suitable tissue culture vessel, such as a bottle, flask or dish, is seeded with CV-IP cells, and the cells are incubated at 37°C in a humidified incubator with an atmosphere of 5-10% $CO_2$ in air, until the cells cover the growth area of the vessel. The cells are harvested by trypsinization and are either seeded into another tissue culture vessel at a dilution of about 1:10, or the cells are counted using a hemacytometer and a specific number of cells are seeded.

An integral component of the inducible promoter cascade system of the present invention is a recombinant host cell line which harbors and expresses DNA encoding the bacterial lac repressor protein. This recombinant host cell line must also harbor DNA which contiguously encodes a promoter of mRNA transcription, the lac operator, and the HIV TAT protein, each in their respective 5′ to 3′ genetic order.

To construct the recombinant host cell line described above, two separate and distinct plasmids were introduced into CV-IP cells. A plasmid known in the art as pCMVlacI [Brown, et al., (1987) Cell 49 pp.603], contains the cytomegalovirus immediate early promoter (CMV IE) transcriptionally linked to the bacterial lac repressor gene. The construction of pCMVlacI has been described, and the plasmid was unaltered prior to its introduction into the CV-IP cells. It is readily apparent to those skilled in the art, that promoters other than CMVIE are suitable for expression of the lac repressor gene in CV-IP and other cell lines. Other suitable promoters include, but are not limited to, SV40 early promoter, SV40 late promoter, mouse mammary tumor virus LTR, rous sarcoma virus LTR, moloney leukemia virus LTR, and herpesvirus thymidine kinase promoter.

It is understood that any recombinant DNA construction or plasmid, which will allow expression of the bacterial lac repressor protein in mammalian or other higher animal cell cultures, is sutiable for use in the present invention in lieu of pCMVlacI.

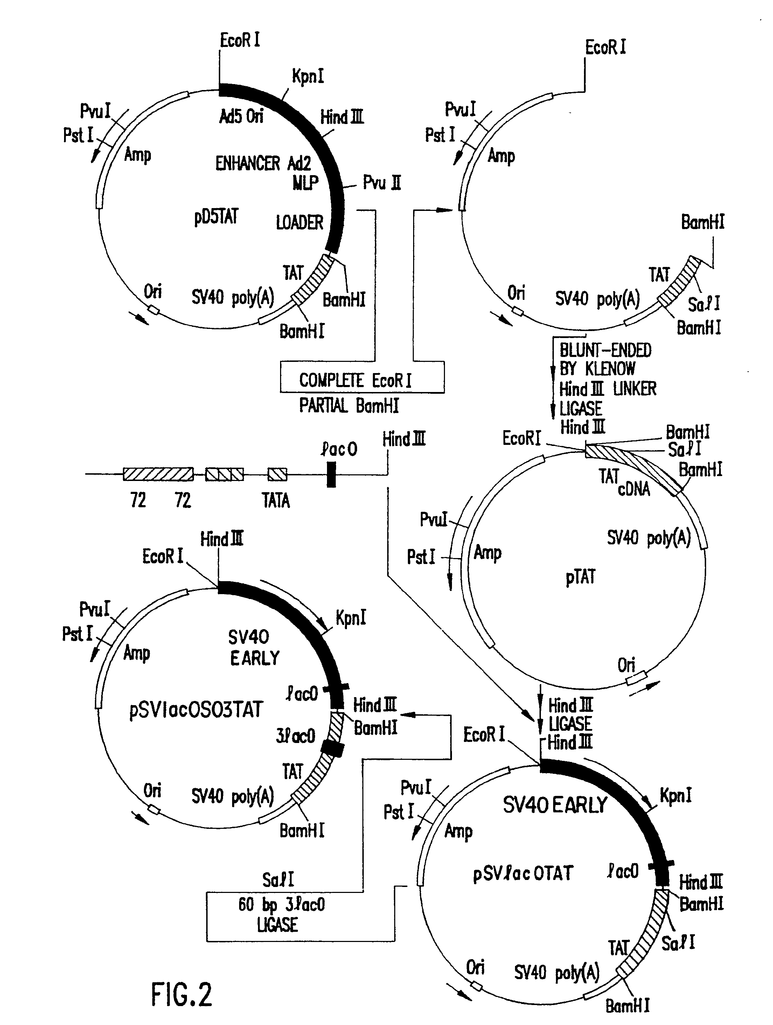

The second plasmid was constructed to contain the SV40 early promoter, multiple lac operator encoding DNA sequences, and the HIV TAT gene, in their respective 5′ to 3′ genetic order Figure 2 illustrates the recombinant DNA cloning strategy set forth below.

DNA encoding the entire TAT protein, linked at its 3′ end with the SV40 late poly-adenylation signal-encoding DNA (SV40 late poly (A)), was obtained from plasmid pD5TAT. pD5TAT is a plasmid known in the art, the construction of which is fully described [Berkner et al., (1988) In Y. Gluzman and S. H. Hughes (ed.) Current Communications in Molecular Biology, Cold Spring Harbor Laboratory, Cold Spring Harbor, NY., pp.56-61; and Arya et al., Science (1985) 229, pp.69]. pD5TAT was digested with the restriction endonuclease EcoRI which cleaves pD5TAT once, resulting in a single, linearized, 4.6 kilobase pair (kbp) DNA molecule. In order to remove the resident transcriptional control elements from pD5TAT, yet maintain the complete TAT-encoding DNA, the EcoRI linearized pD5TAT was partially digested with the restriction endonuclease BamHI. The BamHI partial digestion step does not cleave pD5TAT DNA at both BamHI recognition sites, but instead only cleaves at one BamHI site. As a result, the 3.2 kbp DNA fragment contains the complete TAT gene and the SV40 early poly (A) signal. This 3.2 kbp fragment was purified by preparative agarose gel electrophoresis, and the 5′ phosphates were removed by treatment with calf intestinal alkaline phosphatase (CIAP). The ends of the DNA fragment were then treated with the Klenow fragment of DNA polymerase I, to fill in (blunt-end) the 5′ overhang (sticky-end) resulting from EcoRI and BamHI digestion. The blunt-ended DNA was then ligated to a synthetic DNA oligonucleotide which consists of a HindIII site, forming pTAT. pTAT was amplified by growth in E. coli.

pTAT was digested with HindIII and ligated to a 1.1 kbp DNA fragment derived from pSVlacOCAT. pSVlacOCAT is a plasmid known in the art [Brown et al., Cell (1987) 49, pp.603]. The 1.1 kbp fragment contains the SV40 early promoter and the bacterial lac operator DNA sequence, and was generated by digesting pSVlacOCAT with HindIII. The resulting plasmid was designated pSVlacOTAT.

pSVlacOTAT (4.3 kbp) contains the SV40 early promoter linked to a single copy of the lac operator, which is linked to the TAT gene and SV40 late poly (A) signal. It is understood that it is readily apparent to those skilled in the art, that the SV40 early promoter in pSVlacOTAT can be replaced by other, different promoters, suitable for expression in CV-IP cells and other cell lines. Other suitable promoters include, but are not limited to, cytomegalovirus immediate early promoter, SV40 late promoter, mouse metallothionein promoter, mouse mammary tumor virus LTR, Rous sarcoma virus LTR, moloney leukemia virus LTR and herpesvirus thymidine kinase promoter. It is also readily apparent to those skilled in the art, that the SV40 late poly (A) signal can be replaced by other, different poly (A) signals. Other suitable poly (A) signals include, but are not limited to, SV40 early poly (A), bovine growth hormone poly (A), betaglobin poly (A), and human collagen poly (A).

The purpose of constructing pSVlacOTAT is to provide a plasmid for the expression of TAT protein. In the inducible promoter cascade system of the present invention, it is not desirable to allow constitutive expression of TAT, since this would enable continuous expression of a potentially toxic protein under control of the HIV LTR promoter. The expression of TAT must be tightly controlled in the recombinant host cell. According to the present invention, TAT expression is controlled by the introduction of the lac operator DNA between the SV40 early promoter and the TAT encoding DNA. When the lac repressor protein is present, the repressor protein will bind to the lac operator DNA and prevent TAT expression. There are other genetic mechanisms which function in an analagous manner to the lac repressor/operator system, which can inducibly control gene expression, and are well known in the art. These inducible systems include, but are not limited to, the metallothionein promoter system and the heat-shock response system, which may be substituted for the lac repressor/operator system of the present invention.

It is known in the art that a single copy of the lac operator DNA sequence may not be sufficient to completely repress expression of a downstream gene. This can result in a "leaky" system which, for the purposes of the present invention, would allow some TAT expression, which in turn would disrupt the control over the promoter cascade required to prevent expression of a potentially toxic cloned gene.

To ensure tight, non-leaky control over TAT expression, multiple copies of the lac operator DNA sequence were inserted between the SV40 early promoter and the TAT gene in pSVlacOTAT. Multiple lac operator DNA sequences have a greater probability of binding one or more lac repressor molecules at any given time, due to the dynamic nature of lac repressor interaction with the lac operator. Therefore, multiple lac operators enhance TAT repression. A synthetic 60 base pair DNA oligonucleotide, which contains three tandem copies of the lac operator, was annealed and ligated into the unique SalI site of pSVlacOTAT. The SalI site resides within the 5' noncoding region of the TAT gene. The resulting plasmid was designated pSVlacOSO3TAT. The function of the lac operator sequences, with respect to repression of TAT, is not affected by its precise location between the amino acid-coding portion of the TAT gene and its promoter. Therefore, the use of the SalI site in the present invention, is based on convenience and suitable proximate location, and is not a requirement for TAT repression. It is readily apparent to those skilled in the art that sites other than the SalI site of pSVlacOTAT are suitable sites for the introduction of lac operator sequences. Furthermore, the number of lac operators utilized may be varied, and a plasmid equivalent to pSVlacOSO3TAT does not require the same number of lac operator sequences as pSVlacOSO3TAT to be useful in the present invention.

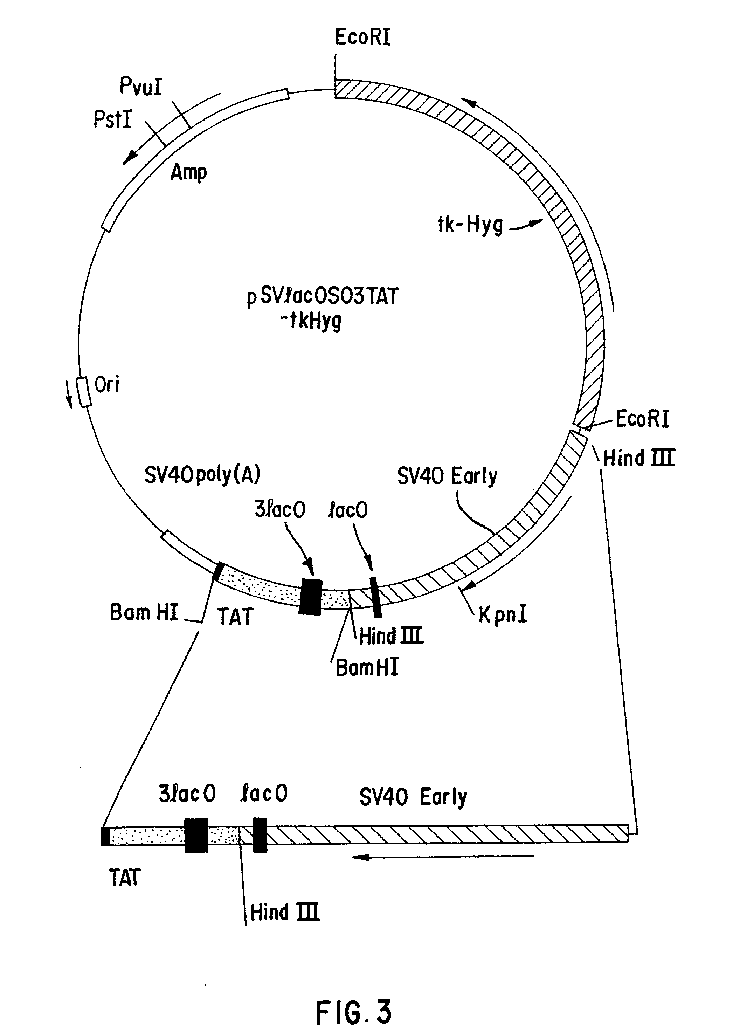

A dominant selectable marker was then cloned into pSVlacOSO3TAT. A HygB$^R$ gene cassette containing the tk promoter, was obtained from pAL2, a poison-minus pBR322 derivative containing the hygromycin B-resistance gene under the control of the Herpesvirus tk promoter. Other plasmids containing a HygB$^R$ gene cassette are commercially available, for example pMAM (catalogue number 6I00-I), Clonetech Labs, Inc., Palo Alto, CA. It is readily apparent to those skilled in the art that all methods of selecting plasmid retaining cells utilized in the present invention are freely interchangeable. Digestion of pAL2 with BamHI removes the 2.0 kbp HygB$^R$ cassette. This 2.0 kbp BamHI fragment was then ligated into the unique BglII site of pSVlacOSO3TAT, forming pSVlacOSO3TAT-tkHyg (see Figure 3).

To obtain a stable cell line which can inducibly express TAT protein, pCMVlacI and pSVlacOSO3TAT-tkHyg were cotransfected into CV-IP cells by the calcium phosphate coprecipitation technique. Selection in medium containing HygB resulted in the emergence of HygB-resistant clones. It is readily apparent to those skilled in the art that other methods of introducing foreign DNA into mammalian cells are suitable for use in the present invention. These methods include, but are not limited to, electroporation, retroviral infection, liposome fusion, DEAE dextran, and microinjection. A solution was prepared for precipitation containing pCMVlacI and pSVlacOSO3TAT-tkHyg, at a molar ratio of 48 to I, respectively. This ratio was used to reduce the number of HygB$^R$ clones containing only plasmid pSVlacOSO3TAT-tk-Hyg. It is not a requirement that both plasmids are simultaneously introduced into the CV-IP cells. In addition, it is not required that any specific molar ratio of plasmids is used.

Individual HygB$^R$ clones were expanded for evaluation of their ability to inducibly express TAT protein, and in turn, express a cloned gene.

For the inducible expression of cloned genes in the promoter cascade system of the present invention, plasmids containing the HIV LTR promoter were constructed. The HIV LTR promoter is transcriptionally activated by TAT protein.

Therefore, a cloned gene transcriptionally linked to HIV LTR will be actively transcribed in the presence of TAT protein. Three plasmids were used, which differ only with respect to the cloned gene linked to the HIV LTR.

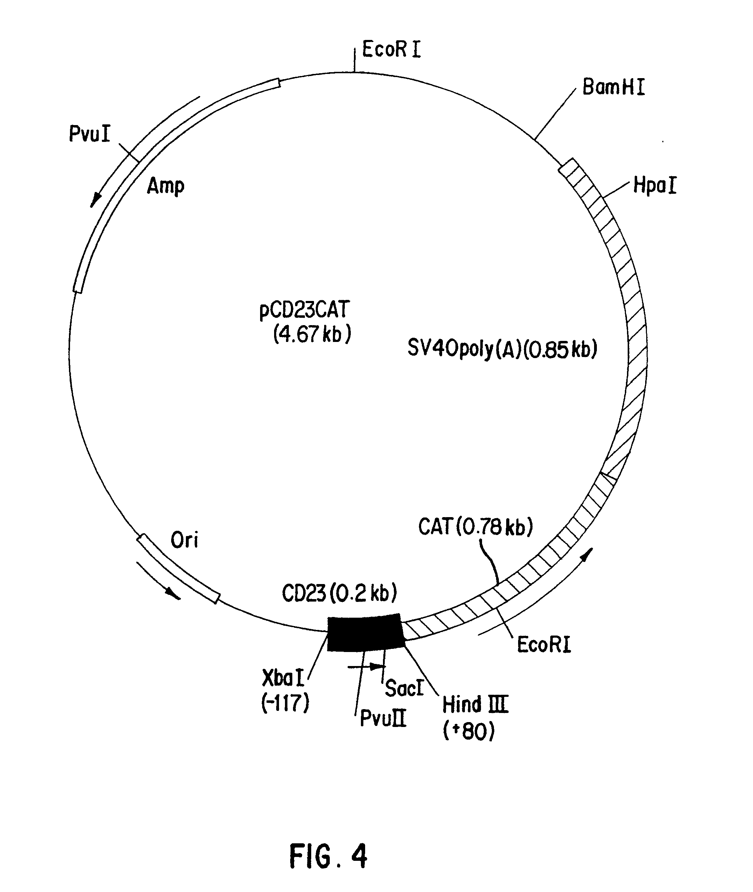

Plasmid pCD23CAT (4.7 kbp), which is known in the art (Siekevitz et al., Science (1987) 238, ppl575), contains a portion of the HIV LTR known as the CD23 promoter (see Figure 4). CD23 encodes the region of the HIV LTR between nucleotides -II7 and +80, with respect to the LTR transcription initiation site. pCD23CAT also contains the chloramphenicol acetyl transferase (CAT) gene transcriptionally linked to the CD23 promoter. CAT activity is easily measurable, and directly correlates with the level of CAT gene expression.

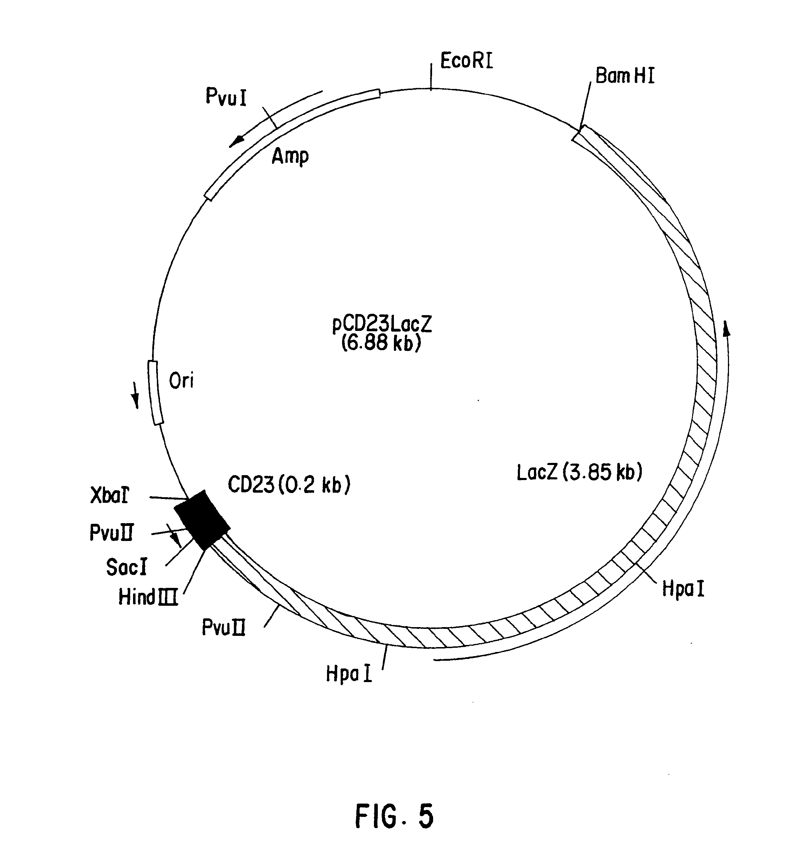

pCD23CAT was digested with HindIII and BamHI to remove the I.6 kbp CAT gene with the SV40 early poly (A) signal, while leaving the remainder of the plasmid intact, which contains the CD23 promoter. The 3.0 kbp CD23-containing fragment was agarose gel-purified and ligated with a 3.8 kbp DNA fragment encoding the beta-galactosidase (Lac Z) gene and the SV40 late poly (A) signal, which was obtained by digestion of pCHII0 (purchased from Pharmacia) with HindIII and BamHI. The resulting plasmid was designated pCD23LacZ (see Figure 5). Beta galactosidase (b-gal) activity is easily measurable and directly correlates with the level of beta galactosidase gene expression.

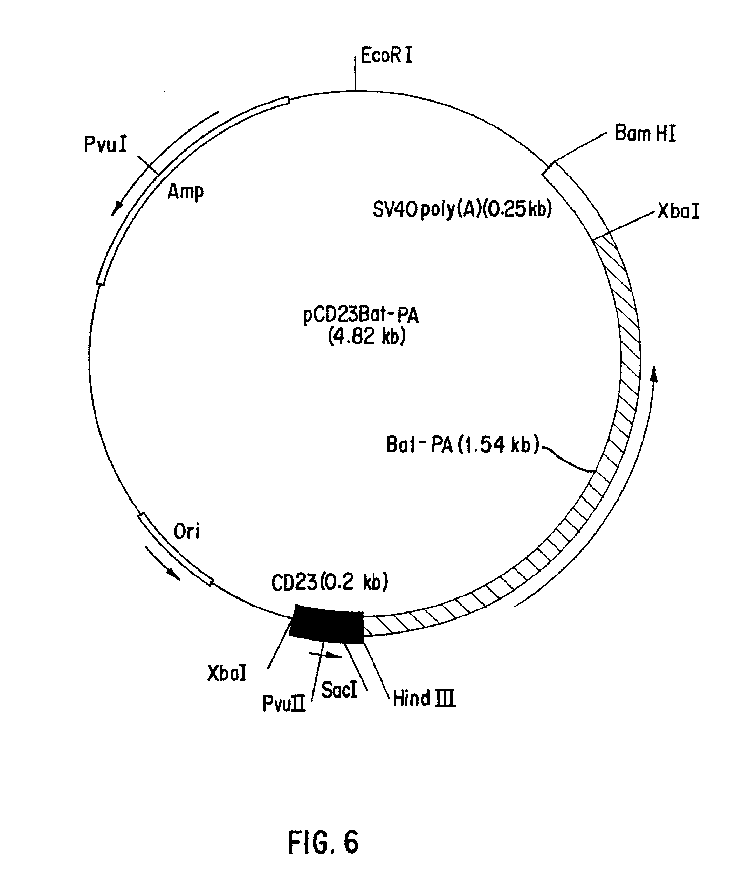

A third plasmid was constructed which contains the CD23 promoter linked to the vampire bat plasminogen-activator (Bat-PA) gene. The 3.0 kbp CD23-containing HindIII- and BamHI-generated fragment of pCD23CAT was ligated with both a I.5 kbp HindIII-and XbaI-generated Bat-PA-encoding DNA fragment, and a 0.25 kbp XbaI- and BamHI-generated DNA fragment encoding SV40 early poly (A) signal. This plasmid was designated pCD23Bat-PA (see Figure 6). Bat-PA activity is easily measured and directly correlates with the level of Bat-PA gene expression.

Each of the expanded HygB[R] clones was screened to determine if the lacI/lacO system was functionally controlling TAT expression. The plasmids pCD23CAT and pCD23LacZ were used for this purpose in transient expression assays. About I x I0[6] cells from an individual HygB[R] clone were seeded into each of three 60 mm tissue culture dishes in tissue culture medium. At the time of seeding, IPTG was added to one of the three dishes of cells at a final concentration of about 20 mM, and all of the plates were incubated for about 24 hours. Two dishes of cells, one with IPTG and one without IPTG, were transfected with either pCD23CAT or pCD23LacZ by the calcium phosphate coprecipitation method. The third dish of cells was mock transfected (no DNA). The cells were then incubated for about 48 hours. CAT assays or b-gal assays were performed on the three dishes of cells from each hygromycin-resistant clone. The dish of cells which was incubated in the presence of IPTG prior to transfection with pCD23CAT exhibited high levels of CAT activity due to derepression of TAT gene expression resulting from the effect of IPTG on the activity of the lac repressor protein. The derepression of TAT expression resulted in the transactivation of the CD23 promoter which, in turn, resulted in high-level expression of the CAT gene.

Conversely, the dish of cells incubated in the absence of IPTG prior to transfection with pCD23CAT exhibited very low levels of CAT activity. In these cells, the expression of TAT was repressed by the lac repressor protein which binds to the lac operator in the absence of IPTG. In the absence of TAT protein the CD23 promoter was not transactivated, which resulted in very low levels of CAT gene expression and CAT activity. The plate of mock-transfected cells was used as a control for background CAT activity endogenously present in the cells.

The same principles hold true for cells transfected with pCD23LacZ. In the presence of IPTG, the cells transfected with pCD23LacZ have high b-gal activity, while cells transfected with pCD23LacZ in the absence of IPTG have very low b-gal activity.

Screening of HygB[R] clones by this method will identify those clones which express the lac repressor protein from pCMVlacI and which contain the TAT gene linked to the SV40 early promoter, which is regulated by the lac operator DNA from pSVlacOSO3TAT-tkHyg. High CAT activity in the cells incubated with IPTG, coupled with low CAT activity in the cells without IPTG and in the control transfected cells, indicates that the lacI/lacO regulation of TAT gene expression is functioning.

From the transient CAT expression assay, HygB[R] clones were identified which exhibited low levels of CAT activity in cells grown without IPTG, and significantly higher levels of CAT activity when IPTG was present. One of the HygB[R] clones exhibited an increase in CAT activity of about 40-fold in the presence of I0 mM IPTG. This HygB[R] clone also exhibited low levels of b-gal activity without IPTG and very high levels of b-gal activity in the presence of IPTG. b-gal activity increased by about 25 fold, 2 days following transfection with pCD23LacZ. This HygB[R] clone was designated CI0TAT (C for CV-IP; I0 for lacI/lacO; and TAT for HIV TAT). Other clones were identified which exhibited similar CAT activities; however, CI0TAT was chosen because of the high ratio of IPTG-induced to uninduced CAT and b-gal expression.

It is understood that clones with lower levels of induced CAT and b-gal activity may be suitable for use in the present invention. It is also understood that clones with high levels of induced CAT or b-gal expression may be suitable for use in the present invention. The choice of CI0TAT does not suggest that the characteristics

6

of this particular HygB$^R$ clone are required for suitability for use in the present invention. It is suggested by the inventors that a minimum level of CAT induction of 10 fold, and a minimum level of b-gal induction of 10 fold, is indicative of suitability for use in the present invention.

Expression of the lac repressor protein in CIOTAT cells was demonstrated by immunoblot analysis of cell lysates. CIOTAT and CV-IP cell lysates were prepared, and immunoblots were performed as described in Example VII. Antibody specific for lac repressor protein identified a protein of 38 kD from the CIOTAT cell lysate which was absent from the CV-IP cell lysate, demonstrating the expression of lac repressor protein in CIOTAT cells.

A sample of CIOTAT cells producing lac repressor protein, and capable of inducing TAT expression in the presence of IPTG, has been deposited without restriction as to availability, in the permanent culture collection of the American Type Culture Collection at 12301 Parklawn Drive, Rockville, MD 20852, and has been assigned the accession number ATCC CRL-10405.

It is readily apparent to those skilled in the art, that effectors (inducers and antiinducers) other than IPTG are suitable for use in the lacI/lacO system. Examples of such effectors include, but are not limited to, methyl b-D-galactoside, methyl
l-thio-b-D-galactoside, n-propyl
l-thio-b-D-galactoside, isopropyl b-D-galactoside,
n-butyl b-D-galactoside, n-butyl
l-thio-b-D-galactoside, benzyl b-D-galactoside,
benzyl l-thio-b-D-galactoside, 2-phenylethyl
b-D-galactoside, 2-phenylethyl l-thio-b-D-galactside,
p-aminophenyl b-D-galactoside, p-aminophenyl
l-thio-b-D-galactoside, O-nitrophenyl
l-thio-b-D-galactoside, p-nitrophenyl
l-thio-b-D-galactoside, D-fucose, galactose,
6-fluoro-6-deoxy-D-galactose, melibiose, and thioallolactose.

CAT activity, as well as b-gal activity, demonstrated the presence of a functional lacI/lacO system regulating TAT gene expression in CIOTAT cells, in a transient CAT and b-gal expression assay. Other genes may be substituted for the CAT gene in pCD23CAT, for transient gene expression in CIOTAT cells. However, it may be more desirable to establish a permanent, stable cell line for the commercial expression of a cloned gene. Permanent, stable cell lines are more amenable to scale up procedures for obtaining large quantities of recombinant proteins and are more easily manipulated in culture, since they do not require a fresh round of DNA transfection to express a cloned gene.

A permanent stable cell line which can inducibly express a cloned gene was established using CIOTAT cells. CIOTAT cells were cotransfected with pCD23Bat-PA and pRSVNeo. The dominant, selectable neomycin-resistance gene is present on pRSVNeo, a plasmid which is known in the art, and is available from the American Type Culture collection, designated ATCC 37198. Both pCD23Bat-PA and pRSVNeo were simultaneously introduced into CIOTAT cells by the calcium phosphate coprecipitation transfection method. A molar ratio of 10 to 1 of pCD23Bat-PA and pRSVNeo, respectively, was used for transfection. It is readily apparent to those skilled in the art, that different molar ratios of the two plasmids will result in a successful transfection of both plasmids into an individual cell. The ratio of 10 to 1 is standard in the art for the purpose of introducing a selectable marker residing on a plasmid independent of a plasmid containing the cloned gene to be expressed.

It is also readily apparent to those skilled in the art, that the neomycin resistance gene, or any other selectable marker, can be directly inserted into pCD23Bat-PA or into any of the CD23 promoter containing plasmids.

Following transfection, the neomycin derivative, G418, was added to the culture medium at about 200 mg/mL. G418-resistant clones emerged which were isolated and expanded, to screen for those clones capable of inducible expression of Bat-PA.

About $2 \times 10^5$ cells were seeded into five wells of a 6-well tissue culture dish (35 mm diameter wells) for each G418-resistant clone. IPTG was then added to the medium of four wells at concentrations of 0.1, 1,5 and 40 mM, with the fifth well receiving no IPTG. A sample of medium was removed from each well following 2, 3, 4, and 6 days of incubation. Since Bat-PA is a secretory protein, the culture medium was directly assayed for Bat-PA activity, using a commercially available (American Diagnostica, Inc.) spectrophotometric plasminogen activator assay.

A G418-resistant clone was identified which inducibly expressed and secreted Bat-PA into the culture medium. Maximum Bat-PA induction was seen (about 100-fold) using between about 1 and 5 mM IPTG, with about 1mM being preferred, after about 6 days of IPTG induction. This G418-resistant clone was designated CIOTAT-Bat-PA. A sample of cell line CIOTAT-Bat-PA has been deposited, without restriction as to availability, in the permanent culture collection of the American Type Culture Collection, at 12301 Parklawn Drive, Rockville,

MD, 20852, and has been assigned the accession number ATCC CRL-l0404.

The plasmids employed in the present invention will, upon introduction into mammalian cells, ultimately integrate into the host cell DNA. The stable cell lines CIOTAT and CIOTAT-Bat-PA contain integrated copies of pCMVlacl, pSVlacOSO3TAT-tkHyg (CIOTAT cells), and pCD23-Bat-PA (CIOTAT-Bat-PA cells). It is readily apparent to those skilled in the art, that other plasmids are suitable as vectors for lac repressor expression (analagous to pCMVlacl), lac operator-regulated TAT expression (analagous to pSVlacOSO3TAT-tkHyg), and any cloned gene expression (analagous to pCD23-Bat-PA). These other suitable plasmid vectors may be of the type which is capable of extrachromosomal replication (episomal), which include but are not limited to, bovine papilloma virus-based vectors, adenovirus-based vectors, and herpesvirus-based vectors. It is also readily apparent to those skilled in the art that marker genes which can be selected for gene amplification and thus act as amplifiable DNA sequences, can be inserted into any of the plasmids employed in the present invention, as well as any of the plasmid vectors which may be substituted for those used in the present invention. Amplifiable DNA sequences which may be inserted into plasmids for use in the inducible promoter cascade of the present invention include but are not limited to, dihydrofolate reductase DNA, multidrug-resistance DNA and glutamine synthetase DNA.

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims and its equivalents.

The following examples illustrate the present invention without, however, limiting the same thereto. The disclosure of each reference mentioned in the following examples is hereby incorporated by reference.

## EXAMPLE I

### Lacl plasmid:

The plasmid pCMVlacl is known in the art, [Brown et al., Cell 49:603 (1987)] and its construction is described as follows. A PstI-generated DNA fragment containing the entire lacIq coding sequence [Calos et al., Mol. Gen. Genet. I83:559 (I98I)] was ligated into the PstI site of pBR322. The initiation codon of the cloned lacI gene was modified from GTG to ATG by replacement with a synthetic oligonucleotide. The resulting DNA, when linked to the b-lactamase promoter, was shown to convert lacI⁻ E. coli to lacI⁺. For expression of lacl in mammalian cells, plasmid pCMVlacl was constructed. This plasmid contains the immediate early gene (IE94) promoter sequence from Simian CMV Colburn strain [Jeang et al., Mol. Cell. Biol.:22I4 (I984)], a I.I kbp DNA fragment containing the intact lacI coding sequence (with the modified ATG codon), and a downstream poly(A) signal.

## EXAMPLE II

### TAT plasmid construction containing the SV40 early promoter and lac operator sequences:

A TAT expression plasmid, pSVlacOTAT, containing the lac operator sequence was constructed as follows. The plasmid pD5TAT containing Adenovirus 5 ori, SV40 enhancer, Adenovirus 2 major late promoter/tripartite leader, TAT cDNA [Arya et al., Science 229 : 69 (I985)] and SV40 late poly-(A) signal [Berkner et al., p. 56-6I. (I988) In Y. Gluzman and S.H. Huges (ed.) Current Communications in Molecular Biology, Cold Spring Harbor Laboratory, Cold Spring Harbor, N.Y.] was digested to completion with restriction endonuclease EcoRI and then partially digested with restriction endonuclease BamHI to remove all regulatory sequences (about I.4 kbp in total) but maintain the TAT cDNA sequence and the SV40 late poly-(A) signal. The resulting 3.2 kbp linearized DNA fragment was treated with CIAP to dephosphorylate the 5′ termini. The termini then were made blunt by treatment with the Klenow fragment of DNA polymerase I, in the presence of all four dNTPs. A phosphorylated HindIII oligonucleotide linker (CCAAGCTTGG) was annealed and then was ligated to the dephosphorylated, blunt-ended fragment. The resulting plasmid (pTAT) was used to transform E. coli for the purpose of preparing a large batch of pTAT.

A I.I kbp SV40 early promoter/,lac operator containing DNA frament was removed from pSVlacOCAT [Brown et al., Cell 49:603 (I987)] by digestion with HindIII, and inserted into the unique linker-generated HindIII site of pTAT to generate the plasmid pSVlacOTAT.

A 60 base pair DNA oligonucleotide containing three tandem copies of the lac operator sequence with an XhoI complementary end:

$$\text{TCGAG}(\text{ATTGTGAGCGCTCACAAT})_3\text{C}$$

was synthesized, annealed and inserted into the unique SalI site (in the 5'-untranslated TAT sequence) of pSVlacOTAT to generate the plasmid pSVlacOSO3TAT (see figure 2).

To construct a pSVlacOSO3TAT plasmid containing a selectable marker, a tk promoter driven HygBR gene was inserted into the unique EcoRI site of pSVlacOSO3TAT as follows. Plasmid pSVlacOSO3TAT was linearized with EcoRI and treated with Klenow fragment to fill in the EcoRI-generated ends, forming blunt ends. The blunt-ended DNA then was dephosphorylated by treatment with CIAP. Phosphorylated BglII linkers (CAGATCTG) then were ligated onto the blunt-ended DNA. The resulting DNA was used to transform E. coli for the purpose of obtaining a sufficient quantity of the TAT plasmid containing the unique BglII site for use in subsequent steps.

A 2.0 kbp tk promoter-driven HygBR gene cassette was prepared from the BamHI digestion of plasmid pAL2, a poison-minus pBR322 derivative containing the hygromycin B-resistance gene under the control of the Herpesvirus tk promoter.. The TAT plasmid containing the unique BglII site then was digested with BglII and ligated with the BamHI-generated HygBR gene cassette. The final construction named pSVlacOSO3TAT-tkHyg (FIG. 3) was used for cotransfection with pCMVlacI to obtain stable cell lines for use in the inducible promoter cascade system.

## EXAMPLE III

### HIVLTR promoter-driven reporter genes:

HIVLTR (or CD23) promoter-driven reporter genes were constructed as follows. The known plasmid pCD23CAT (4.67 kbp) [Siekevitz et al., Science 238:1575 (1987)] is a chloramphenicol acetyl transferase (CAT) gene cassette driven by the CD23 promoter (see Figure 4). The CD23 promoter is a truncated version of the HIV LTR and is encoded between nucleotides -117 and +80, with respect to the HIV LTR transcription initiation site. pCD23 was digested to completion with both HindIII and BamHI to delete the CAT gene and the poly(A) signal. The resulting CD23 promoter-containing plasmid (3.0 kbp) was used for the insertion of other reporter genes.

A 3.8 kbp LacZ expression cassette fragment was removed from plasmid pCHII0 (purchased from Pharmacia) by digestion with HindIII and BamHI and was gel purified. The LacZ gene cassette was ligated into the HindIII- and BamHI-digested CD23 promoter-containing plasmid, generating plasmid pCD23LacZ (see Figure 5).

Another CD23 promoter plasmid was constructed, containing the vampire bat plasminogen activator gene (Bat-PA) [Gardell et al., J. Biol. Chem. 264, pp.17947, (1989)] from plasmid pSZ89-II0-3 [see Duong et al., European Patent Application 352,119, for a full description of pSZ89-II0 and a drawing, Figure I0]. A I.5 kbp Bat-PA gene was prepared by digestion of pSZ89-II0-3 with HindIII and XbaI restriction enzymes. The SV40 early poly(A) signal (0.25 kbp) was obtained from plasmid pRI35 [Rouzer et al., J. Biol. Chem. 263:I0I35 (I988)] by digestion with BamHI and XbaI.

The two DNA fragments encoding Bat-PA and SV40 early poly(A) were both ligated into the HindIII and BamHI digested, CD23 promoter-containing plasmid to generate the construct pCD23Bat-PA (see Figure 6).

## EXAMPLE IV

### Cell Culture

The established African green monkey kidney cell line CV-IP, its HygBR derivative (CIOTAT, aCV-IP clone containing plasmids pCMVlacI, and pSVlacOSO3TAT-tkHyg), and its HygB/G4I8-resistant derivative (CIOTAT-Bat-PA, a CV-LP clone containing plasmids pCMVlacI, pSVlacOSO3TAT-tkHyg, and pCD23Bat-PA) were grown on plastic tissue culture dishes (I00 mm diameter) in Dulbecco's modification of Eagle's minimal essential medium (DME, GIBCO) supplemented with I0% heat-inactivated newborn calf serum (GIBCO). The antibiotic G4I8 (GIBCO) was added to the medium at 400 ug/mL, where indicated.
Hygromycin B (Calbiochem) was added to the medium at 200 ug/mL, where indicated. Cells were cultured in a humidified atmosphere containing I0% $CO_2$ at 37°C.

For the induction of the promoter cascade for high-level gene expression, a stock solution of I M isopropyl b-D-thiogalactoside (IPTG, Promega) was prepared in PBS (GIBCO), and aliquots were added to the cell culture medium to give the appropriate final concentration as needed.

## EXAMPLE V

### DNA transfection

CV-IP cells and lac repressor-producing cells (CIOCAT) [Figge et al., Cell 52:713 (1988)] were grown in DME supplemented with 10% newborn calf serum (NCS) and in medium containing 200 mg/ml G418, respectively. On the day prior to transfection, 1 x 10⁶ cells per 60-mm dish (about 60-80% confluence) were plated. IPTG was added to the culture medium as needed at the time of plating for preincubation.

Supercoiled plasmid DNAs were introduced into CV-IP or CIOCAT cells by the calcium phosphate coprecipitation technique ([Wigler et al., Proc. Nat'l Acad. Sci. USA 76:1373 (1979)]. 0.4 mL of the DNA/CaCl₂ solution was then added dropwise, to 0.4 mL of 2X HBS (HEPES-buffered saline solution, pH 7.05). After 20 to 30 minutes of precipitation at room temperature, 0.8 mL of the precipitate was added to the cells in the 60-mm plates, and the plates were incubated for four hours at 37°C. The medium was removed, and the cells were glycerol-shocked for 3 minutes using 15% glycerol in IX HBS. For transient expression assays, the cells were harvested 48 hours following transfection. For the selection of stable transformants, transfected cells were transferred to medium containing 400 mg/mL of G418, or 200 mg/mL of HygB, three days after transfection. Individual G418- or HygB-resistant colonies were isolated using 10-mm glass cloning cylinders, harvested by trypsinization, and expanded in plastic dishes. Clonal lines were maintained in DME containing 10% NCS and G418 (200 mg/mL) or HygB (100 mg/ml).

## EXAMPLE VI

### CAT Assays

Chloramphenicol acetyl transferase (CAT) assays were performed by a modification of the method of Gorman et al. [Gorman et al., Mol. Cell. Biol. 2:1044 (1982)]. 48 hours after DNA transfection, the plates of cells were washed twice with cold phosphate buffered saline (PBS) and harvested by scraping with a rubber police-man, into 1.2 mL of PBS.

The cell suspension was transferred to a tube and centrifuged at 9,000 Xg for 1 minute. The cell pellets were dispersed in 1 mL of PBS and recentrifuged as before. The cell pellets then were resuspended in 100 ml of 0.25 M Tris-HCl (pH 7.6) by vortexing and then were lysed by sonication or by five serial freeze/thaw cycles. Cellular debris was removed by centrifugation for 15 minutes at 9,000 Xg at 4°C. The protein content of each extract was determined by the BCA protein assay (Pierce) using a standard curve prepared with bovine serum albumin (BSA, Sigma). Aliquots of cell extracts containing equivalent amounts of protein were brought to 50 mL by addition of 0.25 M Tris-HCl (pH 7.6) prior to the CAT assay. Extracts were incubated with 50 mL of a mixture of ¹⁴C-Chloramphenicol (about 100,000 cpm, 54mCi/mmol, Amersham) and acetyl coenzyme A (1.056 mM, lithium salt, Pharmacia) in 0.25 M Tris-HCl for 60 minutes at 37°C. After thin-layer chromatography, the plates were exposed to X-ray film at -80°C. The radioactivity contained in the spots corresponding to chloramphenicol and the acetylated forms of chloramphenicol was determined by liquid scintillation counting. The percentage of acetylated chloramphenicol was calculated by dividing the cpm of the acetylated species of chloramphenicol by the sum of cpm of all forms of chloramphenicol.

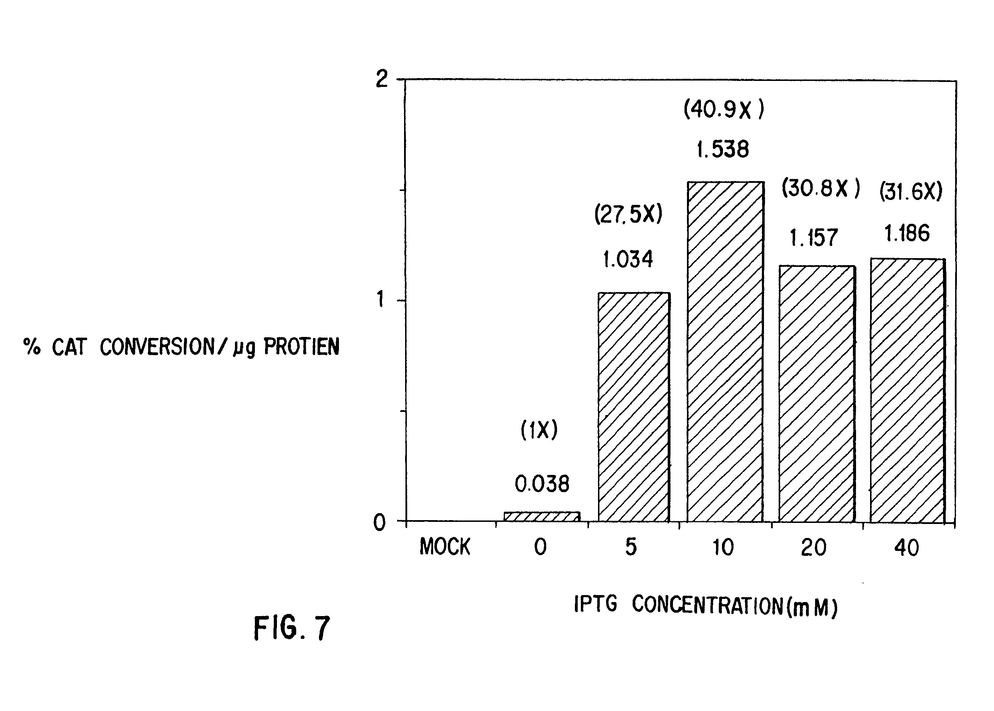

The results of a CAT assay performed on the cell line CIOTAT is shown in Figure 7. A titration of IPTG was performed for the purpose of determining the optimum concentration of IPTG for derepression of TAT gene expression. The concentration of IPTG which caused the greatest increase in CAT activity was determined to be about 10 mM.

## EXAMPLE VII

### Immunoblot Analysius of Lac Repressor

Soluble cytoplasmic extracts were prepared from CIOTAT, CIOCAT and CV-IP cell lines as described previously [Figge et al., Cell 52:713 (1988)]. Cells on confluent 100-mm plates were washed three times with 5 mL of cold PBS, scraped off with 5 mL of PBS, and centrifuged. The cell pellets were resuspended in the appropriate amounts of cold lysis buffer (0.2 M potassium phosphate, pH 7.4, 0.1 mM EDTA, 0.3 mM DTT, 5% (w/v) dextrose, 25 mM leupeptin [Boehringer Mannheim], 1 mg/mL bestatin [Sigma], 10 mg/mL bovine pancrease trypsin inhibitor [Sigma], and 1mM phenylmethylsulfonyl flouride [PMSF, BRL]) to give a cell concentration of 2 x 10⁶ cells/100 mL. The cell suspensions underwent five serial freeze/thaw cycles and were centrifuged to remove cellular debris and nuclei. The supernatant volume was adjusted to have the same protein concentration based on the total protein assay (BCA reagent, Pierce). 20 mL of supernatant was mixed with an equal volume of 2X electrophoresis sample solution (20 mM Tris-HCl, pH 7.0, 5 mM EDTA, 4 % SDS, 20% glycerol, l0% b-mercaptoethanol, 0.5 mg/mL bromophenol blue). Commercially available lacl/Z fusion protein purchased from Promega was dissolved in electrophoresis sample solution and used as a positive control. Proteins in each sample were electrophoresed through precast sodium-dodecyl sulfate (SDS) polyacrylamide gels (l2% or 8-l6% gradient, Schleicher & Schuell) and electrotransferred to nitrocellulose membranes. After transfer, the membrane was washed in TBST (25 mM Tris-HCl, pH 8.0, l25 mM NaCl, 0.05% Tween 20) and incubated in blocking buffer (TBST supplemented with 2% BSA and 0.l% sodium azide) overnight at room temperature. The nitrocellulose membrane then was incubated for 3-5 hours at room temperature with blocking buffer containing 5 mg/mL of mouse anti-lac repressor monoclonal antibody B-2. The nitrocellulose then was washed with blocking buffer lacking antibody. For nonradioactive immunodetection, a l:7500 dilution (in blocking buffer) of a goat anti-mouse IgG alkaline phosphatase conjugate (Promega) then was incubated with the nitrocellulose for 45 minutes at room temperature. The membrane was washed once for 5 minutes with blocking buffer and then rinsed twice with TBST for 5 minutes each. Finally, the membrane was incubated in a color reaction buffer (l00 mM Tris-HCl, pH 9.2, 100 mM NaCl, 5 mM $MgCl_2$) containing l65 mg/mL of 5-bromo-4-chloro-3-indodlyl phosphate (BCIP, Promega) and 330 mg/mL of nitro blue tetrazolium (NBT, Promega). After visible bands were developed, the membrane was rinsed with distilled water to stop the color reaction.

For radioactive immunodetection, a l:l000 dilution (in blocking buffer) of [125]I-labeled sheep anti-mouse IgG (Amersham) was incubated with the membrane for l hour at room temperature. The membrane was washed extensively with TBST four times for 5 minutes each, air-dried and exposed to X-ray film at -80°C.

Both the radioactive and nonradioactive immunodetection methods detected a protein band of about 38 kD which reacted with lac repressor-specific antibody, corresponding with the expected molecular weight and antigenicity of lac repressor protein.

## EXAMPLE VIII

Assay of b-gal activity for screening CIOTAT Stable Clones

The assay for b-galactosidase activity in monolayer cells has been described [Norton and Coffin, Mol. Cell. Biol. 5:28l (l985)]. This spectrophotometric assay was adapted for screening stable CIOTAT transfectants.

After expanding individual HygB[R] colonies, equivalent numbers of cells were seeded in three wells of a 6-well tissue culture dish (35 mm diameter wells). IPTG was added to one well at a final concentration of 20 mM for preincubation. pCD23lacZ plasmid DNA (constructed as described in Example III) was used to make a calcium phosphate precipitate (as described in Example V). Two of the three wells, one without IPTG and one with 20 mM IPTG, were transfected with plasmid pCD23lacZ. One well was mock-transfected and used as a negative control for endogenous b-gal activity and light scattering by cellular debris.

48-72 hours following transfection, the cells were rinsed twice with PBS (GIBCO) and lysed in 0.2 mL of 0.l% SDS in PBS. Then 0.8 mL of Z buffer (60 mM $Na_2HPO_4$, 40 mM $NaH_2PO_4$, l0 mM KCl, l mM $MgSO_4$, 50 mM b-mercaptoethanol; pH 7.0 [Miller, (l972) In Experiments in Molecular Genetics, Cold Spring Harbor Laboratories, Cold Spring Harbor, N.Y.]) was added. The samples were vortexed and incubated at 37°C until a yellow color was visible.

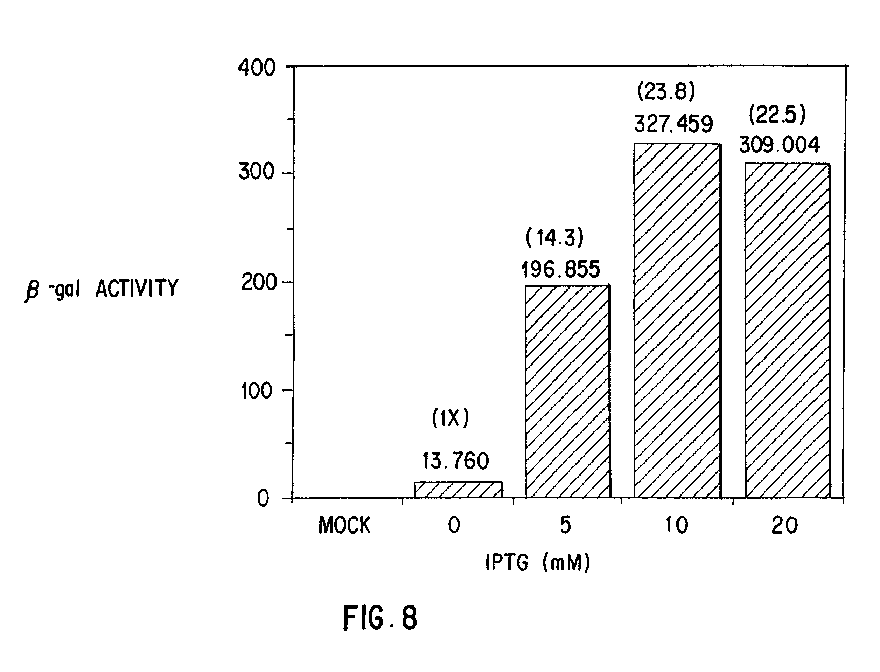

The tubes then were placed on ice and reactions were terminated by adding 0.5 mL of l.0 M $Na_2CO_3$. The reaction time (in minutes) and the absorbance at 420nm were used to calculate specific b-gal activity. The results of a b-gal assay performed on the cell line CIOTAT is shown in Figure 8. A titration of IPTG was performed for the purpose of determining the optimal IPTG concentration which induced the greatest increase in b-gal activity, and was determined to be about l0 mM.

## EXAMPLE IX

Spectrolyse assay of bat plasminopen activator

Cells which harbor and coexpress all three plasmids pCMVlacl, pSVlacOSO3TAT and pCD23Bat-PA, were plated in 6 well microtiter dishes at 2xl0[5] cells per well, in medium with containing various concentrations of IPTG. Aliquots of medium were collected after two, three, four and six days of IPTG induction and were assayed for Bat-PA activity using the Spectrolyse t-PA assay kit (American Diagnostica, Inc.) modified according to Gardell et al. [J. Biol. Chem. 264, in press (l989)]. Plasminogen activator was incubated with human Glu-plasminogen (40 mg/ml), Spectrozyme PL (0.4 mM) and DESAFIB (80 mg/ml) in 0.l M Tris-HCl, and 0.l% Tween 80; pH 8.0. The reactions were carried out in 96-well microtitration plates, for 30 minutes at room temperature, and the release of free p-nitroaniline from the chromogenic substrate was measured spectrophotometrically using a BioRad kinetic reader (model 3550).

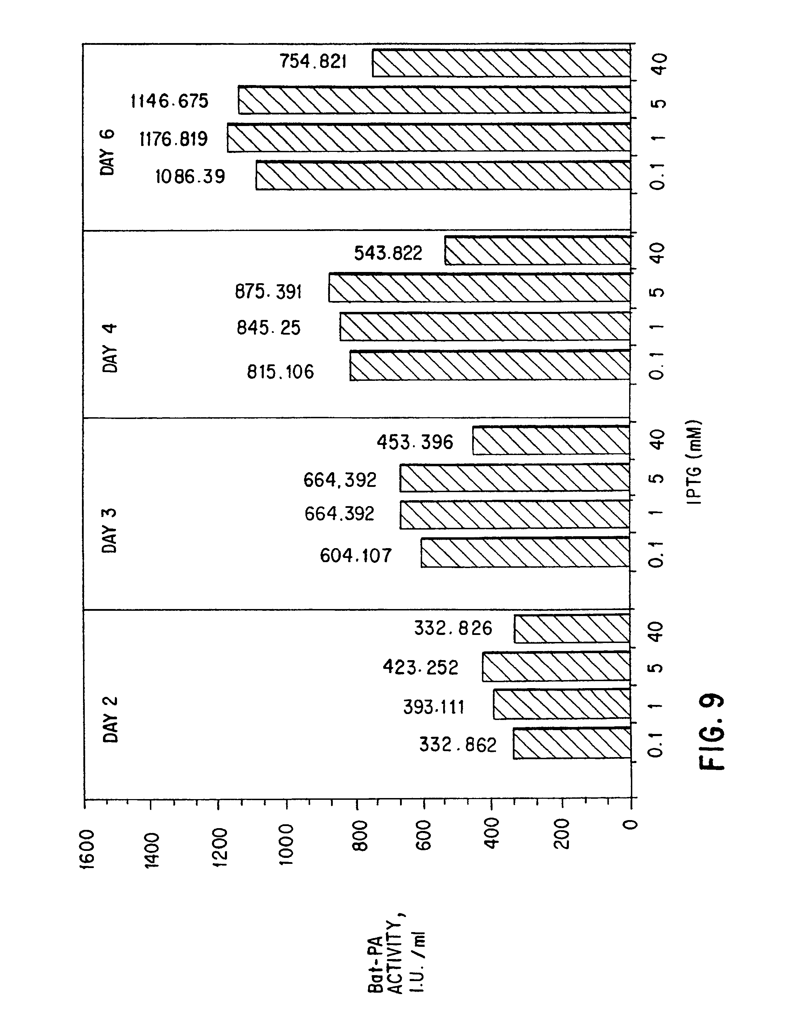

The Bat-PA activity (International Units/ml) was determined by comparing the increase in $A_{405}$ of the sample, with a standard t-PA activity curve. The results are shown in Figure 9. A titration of IPTG demonstrated that I-5 mM IPTG resulted in maximal Bat-PA activity. After six days of I mM IPTG induction, Bat-PA activity was increased at least 80-fold to about 2.5mg/mL, based on 500 International Units/mL equivalent to Img/mL. This demonstrates the ability of IPTG to induce TAT gene expression, which transactivates the CD23 promoter of pCD23 Bat-PA, resulting in the induction of high levels of Bat-PA expression, while keeping the levels of Bat-PA low before IPTG induction.

## Claims

1. A plasmid which contains DNA encoding HIV TAT protein which is transcriptionally regulated by DNA encoding the lac operator.

2. A plasmid containing HIV LTR promoter DNA controlling transcription of a cloned foreign gene, which can be trans-activated by TAT protein, for use in an inducible gene expression system.

3. The plasmid according to Claim 2, in which the cloned foreign gene consists of DNA encoding bat plasminogen activator.

4. A recombinant mammalian cell line which expresses the lac repressor protein and contains DNA encoding HIV TAT protein which is transcriptionally regulated by DNA encoding the lac operator.

5. A recombinant mammalian cell line which:
   a) expresses the lac repressor protein;
   b) contains DNA encoding HIVTAT protein which is regulated by DNA encoding the lac operator; and
   c) contains DNA comprising the HIV LTR promoter controlling transcription of DNA encoding bat plasminogen activator, which can be trans-activated by TAT protein.

6. An inducible gene expression system for the controlled expression of cloned genes in mammalian cells comprising:
   a) a recombinant mammalian cell line which expresses a transcription repressor protein, and contains DNA encoding a transactivator protein which is regulated by DNA encoding the target for the transcription repressor protein,
   b) a plasmid introduced into said recombinant cell line, said plasmid containing a promoter controlling transcription of the cloned foreign gene, which is capable of transactivation by the transactivator protein, resulting in an inducible gene expression system,
   c) said system being inducible for expression of the cloned gene by addition of an inducer, which inducer causes the derepression of the transactivator protein expression and subsequent trans-activation of the transactivatable promoter of (b).

7. An inducible gene expression system for the controlled expression of cloned genes in mammalian cells comprising:
   a) a recombinant mammalian cell line which expresses lac repressor protein, and contains DNA encoding a transactivator protein which is regulated by DNA encoding the lac operator,
   b) a plasmid introduced into said recombinant cell line, said plasmid containing a promoter controlling transcription of the cloned foreign gene, which is capable of transactivation by the transactivator protein, resulting in an inducible gene expression system,
   c) said system being inducible for expression of the cloned gene by addition of a chemical inducer, which inducer causes the derepression of the transactivator protein expression and subsequent trans-activation of the transactivatable promoter of (b).

8. An inducible gene expression system for the controlled expression of cloned genes in mammalian cells comprising:
   a) a recombinant mammalian cell line which expresses lac repressor protein, and contains DNA encoding HIVTAT protein which is regulated by DNA encoding the lac operator,

b) a plasmid introduced into said recombinant cell line, said plasmid containing HIV LTR promoter controlling transcription of the cloned foreign gene, which is capable of transactivation by TAT protein,resulting in an inducible gene expression system,

c) said system being inducible for expression of the cloned gene by addition of a chemical inducer, which inducer causes the derepression of TAT expression and subsequent trans-activation of the HIV LTR promoter.

9. The recombinant mammalian cell line according to Claim 3, with the ATCC deposit accession number CRL-l0404.

10. The recombinant mammalian cell line according to Claim 4, with the ATCC deposit accession number CRL-l0405.

REPRESSION (OFF)                    DEPRESSION (ON)

| CMVIE | *lac* I |                | CMVIE | *lac* I |

REPRESSOR                          REPRESSOR

OO                                 OO  ← I PTG
OO                                 OO

                                   X

| SV40E | TAT |                    | SV40E | TAT |
*lac* O                            *lac* O

NO   TAT                           TAT

| HIVLTR | Bat - PA |              | HIVLTR | Bat - PA |

(CD23)                             (CD23)

NO  Bat - PA                       HIGH  Bat - PA

FIG. 1

FIG.2

FIG. 3

FIG. 4

FIG. 5

FIG. 6

FIG. 7

FIG. 8

FIG.9